# EUROPÄISCHE PATENTSCHRIFT

(11) **EP 1 498 395 B1**
(45) Veröffentlichungstag und Bekanntmachung des Hinweises auf die Patenterteilung: **10.10.2007**
(21) Anmeldenummer: 04103271.5
(22) Anmeldetag: 09.07.2004
(51) Int. Cl.: C03C 4/00, C03C 3/00

(54) **Verwendung von Glaszusammensetzungen zum Erzielen eines antioxidativen Effektes**
Use of a glass composition with the aim of attaining an antioxidative effect
Utilisation d'une composition vitreuse pour obtenir un effet antioxydant

(30) Priorität: 14.07.2003 DE 10332011
(43) Veröffentlichungstag der Anmeldung: 19.01.2005
(73) Patentinhaber: Schott AG, 55122 Mainz (DE)
(72) Erfinder: Fechner, Jörg Hinrich, Dr., 55118, Mainz (DE); Zimmer, José, 55218, Ingelheim (DE); Lee, Sean, Dr., 76224, Karlsruhe (DE)
(74) Vertreter: Fuchs

(56) Entgegenhaltungen:
- WO-A-01/03650
- WO-A-01/72262
- WO-A-03/050052
- WO-A-03/050053
- DE-A- 10 141 230
- US-A- 5 019 293
- US-A1- 2003 008 759
- US-B1- 6 475 631
- DATABASE WPI Section Ch, Week 199322 Derwent Publications Ltd., London, GB; Class L01, AN 1993-182424 XP002299261 & WO 93/10058 A1 (GRAPHITE-BASED CONSTR MATERIALS RES INST) 27. Mai 1993 (1993-05-27)
- PATENT ABSTRACTS OF JAPAN Bd. 2000, Nr. 10, 17. November 2000 (2000-11-17) -& JP 2000 191339 A (KOA GLASS KK), 11. Juli 2000 (2000-07-11)

## Beschreibung

Die Erfindung betrifft die Verwendung von Glaszusammensetzungen in der Form von Glaspulvern, Fasern, Granulaten, Kugeln zum Erzielen eines antioxidativen Effektes.

Die erfindungsgemäßen Glaszusammensetzungen können ebenfalls durch einen Temperschritt in Glaskeramiken mit unterschiedlichem Kristallisationsgrad umgewandelt und als Antioxidantinen verwendet werden. Ebenfalls kann eine vollständige Umwandlung in eine Keramik durchgeführt werden.

Dabei kann die Glaszusammensetzung vielseitig verwendet werden, wie in Kosmetikprodukten (u.a. "Anti-Aging" Produkte), Medizinprodukten, Lebensmitteln und Futtermitteln, Farben und Lacken, und in Polymeren.

Hierbei kann der Einsatz der hier beschriebenen Materialien einerseits zum Schutz des Produktes selbst erfolgen, anderseits aber auch um eine antioxidative Wirkung nach außen zu erzielen (z.B. in Nahrungsergänzungsstoffen)

Während die Verwendung von antimikrobiell und oder biozid wirkenden Gläsern aus einer Vielzahl von Schriften bekannt sind, ist der Einsatz von Glaszusammensetzungen, um Oxidationsprozess zu stoppen oder zu verlangsamen, bislang vollständig unbekannt gewesen.

Durch Verwendung von Antioxidationsmitteln können Oxidationsprozesse gestoppt bzw. verlangsamt werden, die einen negativen Effekt auf verschiedene Produkte haben. So kann zum Beispiel das Auftreten von freien Radikalen in den oben genannten Produkten eine ungewünschte Zersetzung, Verfärbung oder gar die vollständige Unbrauchbarkeit des Produktes zur Folge haben.

Antioxidationsmittel demnach auch produktkonservierende oder sogar produkterhaltende Eigenschaften aufweisen.

Oxidationseffekte sind u.a. auch für die Alterung der Haut verantwortlich ("Aging"). Diese Oxidationen, bzw. Bildung von Radikalen werden u.a. durch UV-Strahlung des Sonnenlichts bzw. auch durch Schadstoffe in der Luft verursacht. Diese Effekte sind u.a. auch für eine Faltenbildung verantwortlich. Daher finden Antioxidationsmittel auch in Kosmetikprodukten Anwendung, die einer vorzeitigen Hautalterung vorbeugen sollen (so genannte Antifaltenmittel).

Bei den bisher beschrieben und bekannten Antioxidationsmitteln handelt es sich fast ausschließlich um organische Verbindungen. Dem Fachmann bestens bekannt sind unter anderem Vitamin C (Ascorbinsäure) und auch Vitamin E. Diese Verbindungen weisen zum Teil den Nachteil auf, dass sie selbst nicht photostabil sind, d.h. dass sie als Radikalbildner wirken können. Diesen Nachteil weist u.a. das bekannte Oxidationsmittel Vitamin C auf, insbesondere wenn es in kleinen Konzentrationen eingesetzt wird.

Es besteht somit ein Bedürfnis, ein Antioxidationsmittel bereitzustellen, das für die oben genannten Anwendungen eingesetzt werden kann und das die Nachteile der bekannten Antioxidationsmittel nicht aufweist.

Überraschenderweise ist nun gefunden worden, dass Glaszusammensetzungen, wie in den Patentansprüchen definiert, zum Zwecke des Vermeidens von Oxidationsvorgängen, also als Antioxidationsmittel, einsetzbar sind. Die Glaszusammensetzungen können oxidisch sein.

Die erfindungsgemäßen antioxidativen Zusammensetzungen weisen eine hohe Photo- und Temperaturstabilität auf. Vorzugsweise wirken die antioxidativen Glaszusammensetzungen biozid auf Bakterien, Pilzen und Viren oder sind biostatisch. Weiter bevorzugt sind sie beim Kontakt mit dem Menschen hautverträglich und sogar zum Verzehr geeignet, dazu toxikologisch unbedenklich und umweltverträglich. Die Zusammensetzungen sind folglich vorzugsweise frei von Schwermetallen, insbesondere wenn diese mit Lebewesen in direkten Kontakt kommen.

Die hierbei verwendeten Glaszusammensetzungen umfassen Glaskeramiken, Fasern, Granulate, Kugeln und Glaspulver. Ein Glaspulver kann ein Pulver sein, das eine Vielzahl von Glaspartikeln in beliebiger Form (auch Fasern) enthält, beispielsweise Glaskugeln mit einer Partikelgröße (d50) von kleiner als 1 mm bis zu kleiner als 500 µm, besonders bevorzugt < 100µm und <20µm, insbesondere <10µm und <5µm oder Glasfasern mit einem Durchmesser von kleiner als 1 mm bis zu kleiner als 500 µm besonders bevorzugt < 100µm und <20µm, insbesondere <10µm und <5µm.

Erfindungsgemäße Glaskeramiken können durch Tempern hergestellt werden und können unterschiedliche Kristallisationsgrade aufweisen.

Die antioxidative Wirkung kann auf einer Reaktion des Glases mit umgebenden Substanzen beruhen.

Reaktionen an der Glasoberfläche können ebenfalls zur antioxidativen Wirkung beitragen. Es wird vermutet, dass die Zusammensetzungen auf verschiedene Weise den antioxidativen Effekt bewirken, zum Beispiel durch das Abfangen freier Radikale, aber auch durch das Unterbrechen von Reaktionsketten, die für die Bildung freier Radikale und / oder Oxidationsprozessen verantwortlich sind und/oder das Ausüben eines Chelat-Effektes.

Die Erfindung wird anhand der folgenden Ausführungsbeispiele näher beschrieben, welche der Erläuterung dienen:

### Beispiel 1:

Aus den in Tabelle 1 gezeigten Rohstoffen wurden Gläser erschmolzen, die anschließend zu Ribbons geformt wurden.

Diese Robbons wurden mittels Trockenmahlung zu Pulver mit einer Partikelgröße d50 = 4µm weiterverarbeitet.

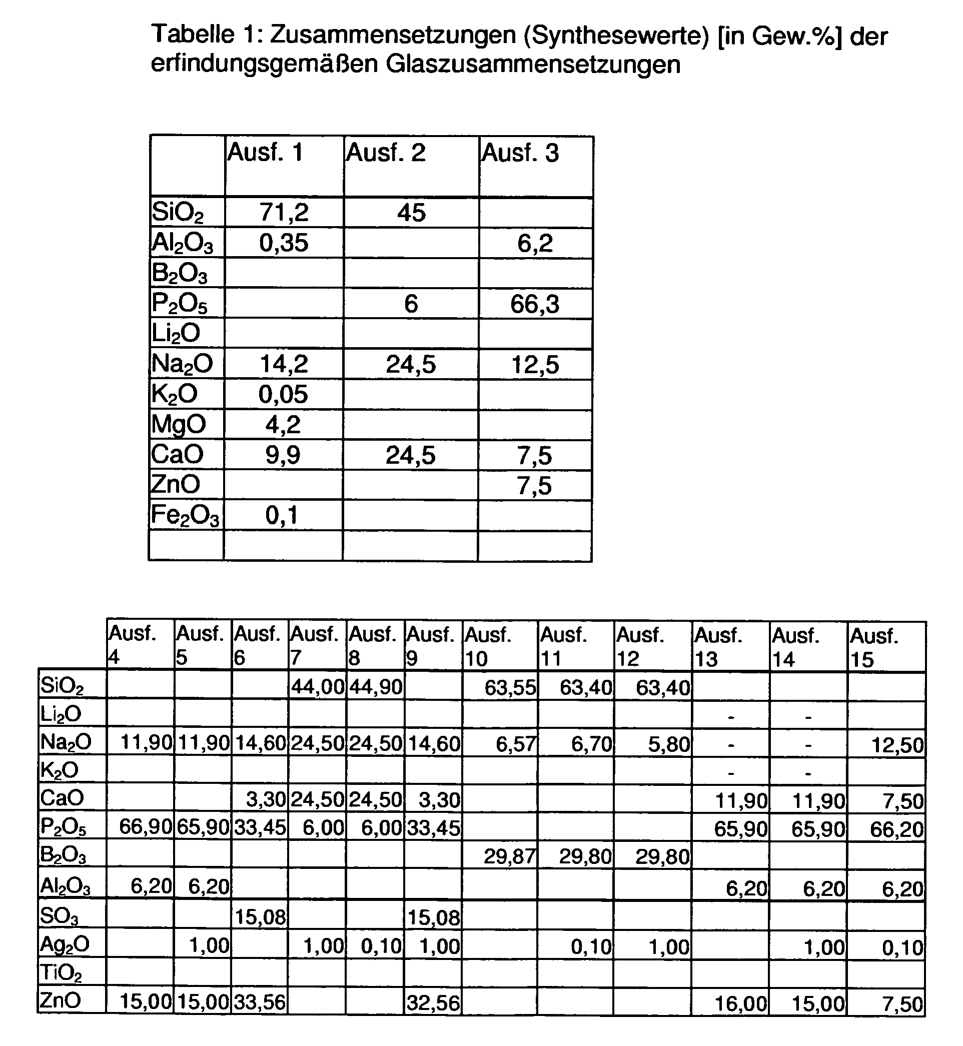

Die antioxidative Wirksamkeit wurde mittels des so genannten Desoxyribose-Tests ("The Desoxyribose Method", Analytical Biochemistry, 165, 215-219, 1987) nachgewiesen.

Die folgende Graphik zeigt den Hydroxylradikal inhibierenden Effekt der Ausf. 3 in Abhängigkeit von der Konzentration:

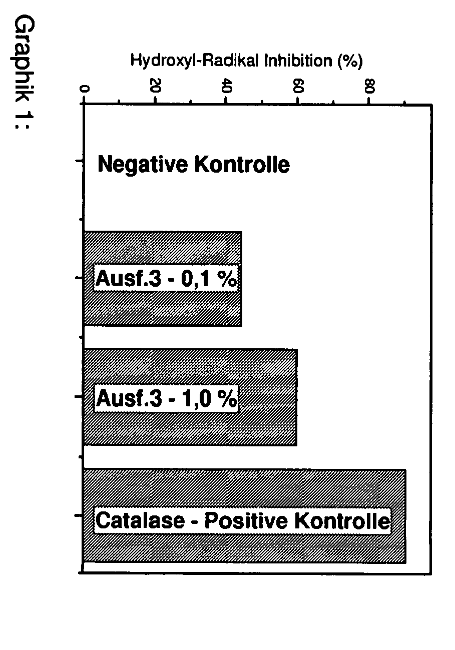

Aus Graphik 1 wird ersichtlich, dass Ausf. 3 einen deutlichen Hydroxylradikal inhibierenden Effekt bewirkt, welcher mit steigender Konzentration zunimmt.

Es kann gezeigt werden, dass die antioxidative Wirkung auf einer Unterbrechung der Redox-Reaktionskette beruhen kann. In dem hier beschriebenen Testverfahren beruht die Wirkung auf einer Chelatbildung (Komplexierung) des für den Bildungsprozess der freien Radikale notwendigen Eisens. Somit wird der Fe⁽²⁺⁾/Fe⁽³⁺⁾ Red-Ox Prozess unterbrochen.

### Beispiel 2:

Ein alternatives Testverfahrens zum Nachweis des antioxidativen Effekts kann mittels des so genannten "Protein-Carbonyl-Assay-Tests", beschrieben in "R.L. Levine et al, Meth. Enzymol. 186, 464-478, 1990", durchgeführt werden.

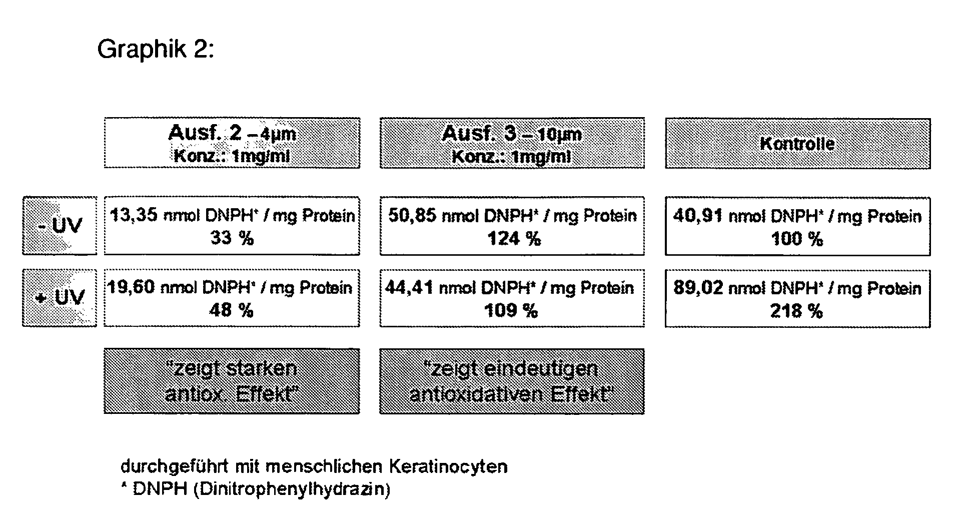

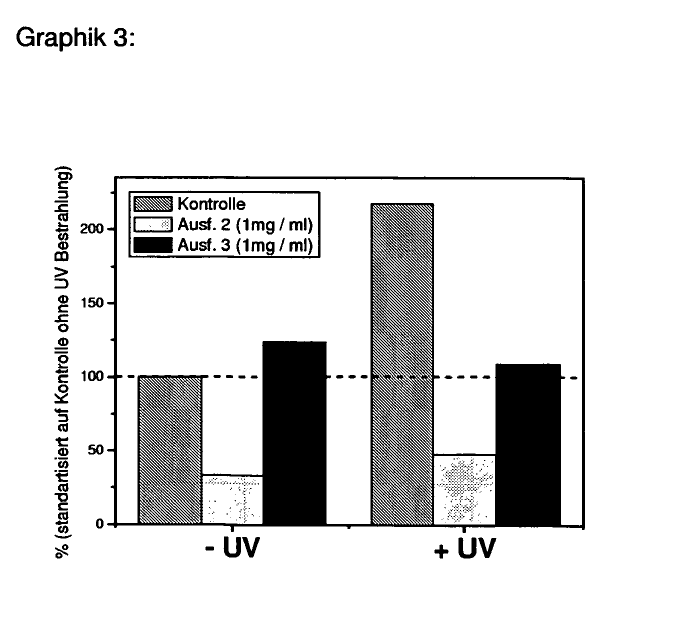

Graphik 3 zeigt das Maß des antioxidativen Effektes anhand erhaltener, oxidierter Proteine. Der Test wurde vor der Bestrahlung mit UV-Licht (-UV) und nach UV-Licht Einwirkung durchgeführt (+UV).

Je niedriger der Balken in Graphik 3, desto größer ist die antioxidative Wirkung für die jeweilige Zusammensetzung.

Die Balkengraphik zeigt, dass mit Ausf. 2 nur sehr wenig oxidierte Proteine erhalten wurden, d.h., dass der antioxidative Effekt groß war.

Auch Ausf. 3 zeigte nach UV-Bestrahlung einen deutlichen antioxidativen Effekt.

### Beispiel 3:

Deoxiribose-Test zur Bestimmung der antioxidativen Wirkung von Shampoos, die erfindungsgemäße Glaszusammensetzungen enthalten.

**Tabelle 2:**

| Gemessene optische Dichten und daraus berechnete Inhibition | | | | |
|---|---|---|---|---|
| **Probe/Konzentration** | **Optische Dichte bei 352 nm 1 % des Shampoos** | **% Inhibition** | **Optische Dichte bei 352 nm 10% des Shampoos** | **% Inhibition** |
| Shampoo 1 mit 5% Glaszusammensetzung (Ausf. 2) | 0,3505 | nicht bestimmbar | 0,2922 | 15,5 |
| Shampoo 2 mit 5% Glaszusammensetzung (Ausf. 3) | 0,2426 | 29,9 | 0,2477 | 28,4 |
| Shampoo 3 keine Glaszusammensetzung (Träger) | 0,3808 | nicht bestimmbar | 0,3166 | 8,5 |
| Katalase (positive Kontrolle) | 0,0471 | 86,4 | nicht anwendbar | nicht anwendbar |
| Referenz (negative Kontrolle) | 0,3459 | nicht anwendbar | nicht anwendbar | nicht anwendbar |

Aus der obigen Tabelle wird ersichtlich, dass das Shampoo, das die Glaszusammensetzung gemäß Ausf. 2 enthält, einen leichten antioxidativen Effekt zeigt, wenn die Testkonzentration des getesteten Shampoos 10% beträgt.

In dem durchgeführten Test kann für das Shampoo, das die Glaszusammensetzung gemäß Ausf. 3 enthält, ein etwa 30%iger Abbau der Deoxyribose durch Hydroxyl-Radikale bestimmt werden, was einem ausgeprägten antioxidativen Effekt entspricht.

Der Träger selbst (Shampoo 3, ohne Glaszusammensetzung) zeigt mit einer Inhibition von 8,5% einen leichten antioxidativen Effekt.

Die Shampoos 1 bis 3 weisen die folgende Zusammensetzung auf (Tabelle 3):

| | | | **Shampoo 1** | **Shampoo 3** | **Shampoo 2 (Träger)** |
|---|---|---|---|---|---|
| **Nr.** | **Rohmaterial** | **INCI*** | **[Gew.%]** | **[Gew.%]** | **[Gew.%]** |
| 1 | Texapon NSO | Sodium Laureth Sulfate | 50,00 | 50,00 | 50,00 |
| 2 | Amphotensid B4 | Cocamidopropy I Betaine | 8,0 | 8,0 | 8,0 |
| 3 | Wasser, demineralisiert | Wasser | 33,29 | 38,29 | 33,29 |
| 4 | Euxyl K40() | Methyldibromo Glutaronitril und Phenoxyethanol | 0,1 | 0,1 | 0,1 |
| 5 | Edeta BD | Dinatrium EDTA | 0,1 | 0,1 | 0,1 |
| 6 | Veegum Ultra | Magnesium Aluminium Silikat | 2,0 | 2,0 | 2,0 |
| 7 | Propylene Karbonat | Propylene Karbonat | 0,01 | 0,01 | 0,01 |
| 8 | Glaszusammensetzung nach Ausf. 2 | | 5,0 | - | - |
| 9 | Glaszusammensetzung nach Ausf. 3 | | - | - | 5,0 |
| 10 | NaCl | Natriumchlorid | 1,5 | 1,5 | 1,5 |
| | | | 100,00 | 100,00 | 100,00 |
| | pH-Wert | | 10,2 | 5,7 | 5,1 |

| | | | | | |
|---|---|---|---|---|---|
| *** international nomenclature of cosmetic ingredients** | | | | | |

### Beispiel 4:

Deoxiribose-Test zur Bestimmung der antioxidativen Wirkung von OW-Formulierungen, die erfindungsgemäße Glaszusammensetzungen enthalten.

**Tabelle 4:**

| Gemessene optische Dichten und daraus berechnete Inhibition | | | | |
|---|---|---|---|---|
| **Probe/Konzentration** | **Optische Dichte bei 352 nm 1% der OW-Formulerung** | **% Inhibition** | **Optische Dichte bei 352 nm 10% der OW-Formulerung** | **% Inhibition** |
| OW-Formulierung 1 mit Glaszusammensetzung (Ausf. 2) | 0,3986 | nicht bestimmbar | 0,3798 | 4,1 |
| OW-Formulierung 2 mit Glaszusammensetzung (Ausf. 3) | 0,2505 | 36,8 | 0,2039 | 48,5 |
| Katalase (positive Kontrolle) | 0,0829 | 79,1 | nicht anwendbar | nicht anwendbar |
| Referenz (negative Kontrolle) | 0,3962 | nicht anwendbar | nicht anwendbar | nicht anwendbar |

Aus der obigen Tabelle wird ersichtlich, dass die OW-Formulierung, die die Glaszusammensetzung gemäß Ausf. 2 enthält, einen leichten antioxidativen Effekt zeigt, wenn die Testkonzentration der getesteten Formulierung 10% beträgt.

In dem durchgeführten Test kann für die OW-Formulierung, die die Glaszusammensetzung gemäß Ausf. 3 enthält, ein etwa 37%iger, bzw. 49%iger Abbau der Deoxyribose durch Hydroxyl-Radikale bestimmt werden, was einem ausgeprägten antioxidativen Effekt entspricht.

Die OW-Formulierungen 1 und 2 weisen die folgenden Zusammensetzungen auf:

**(Tabelle 5):**

| | | **OW-Formulierung 1** | **OW-Formulierung 2** |
|---|---|---|---|
| **Nr.** | **Rohmaterial** | **[Gew.%]** | **[Gew.%]** |
| 1 | Alacel 165V | 6,65 | 6,65 |
| 2 | Cetylalkohol (Lanette 16) | 0,95 | 0,95 |
| 3 | Stearingsäure L2 SM (GV S052) | 4,75 | 4,75 |
| 4 | Neutralöl pflanzl./ Crodamol GTCC (GV N020) | 22,8 | 22,8 |
| 5 | demineralisiertes Wasser | 59,85 | 59,85 |
| 6 | Glaszusammensetzung nach Ausf. 2 | 5,0 | - |
| 7 | Glaszusammensetzung nach Ausf. 3 | - | 5,0 |

## Patentansprüche

1. Verwendung einer Glaszusammensetzung zum Erzielen eines antioxidativen Effektes, im Sinne eines Radikalfängers oder einer antioxidanz.

2. Verwendung nach Anspruch 1, wobei die Glaszusammensetzung oxidisch ist.

3. Verwendung nach einem der vorhergehenden Ansprüche, wobei die Glaszusammensetzung eine hohe Photo- und Temperaturstabilität aufweist.

4. Verwendung nach einem der vorhergehenden Ansprüche, wobei die Glaszusammensetzung folgende Inhaltsstoffe aufweist
SiO₂ 0 - 80 Gew.%
P₂O₅ 0 - 80 Gew.%
SO₃ 0 - 40 Gew%
B₂O₃ 0 - 80 Gew.%
Al₂O₃ 0 - 20 Gew.%
Li₂O 0 - 30 Gew%
Na₂O 0 - 40 Gew.%
K₂O 0 - 30 Gew.%
CaO 0 - 25 Gew.%
MgO 0-15 Gew.%
SrO 0 - 15 Gew.%
BaO 0 - 15 Gew%
ZnO 0 - 40 Gew.%
Ag₂O 0 - 5 Gew.%
F 0 - 10 Gew.%
J 0 - 10 Gew.%
Fe₂O₃ 0 - 5 Gew.%,
und gegebenenfalls Spurenelemente und/oder übliche Läutermittel in gängigen Mengen,
wobei die Summe von SiO₂ + P₂O₅ + SO₃ + B₂O₃ + Al₂O₃ größer als 20 Gew.% und bis zu 80 Gew.% ist.

5. Verwendung nach einem der vorhergehenden Ansprüche, wobei die Glaszusammensetzung frei von Alkalioxiden ist.

6. Verwendung nach einem der vorhergehenden Ansprüche, wobei in der Glaszusammensetzung die Summe von CaO + MgO + SrO + BaO + ZnO von 5 bis 50 Gew.% beträgt.

7. Verwendung nach einem der Ansprüche 1 bis 4 oder 6, wobei in der Glaszusammensetzung die Summe von Li₂O + Na₂O +K₂O von 5 bis 60 Gew.% beträgt.

8. Verwendung nach einem der vorhergehenden Ansprüche, wobei die Glaszusammensetzung folgende Inhaltsstoffe aufweist
P₂O₅ 1 - 15 Gew.%
B₂O₃ 0 - 5 Gew.%
Al₂O₃ 0 - 10 Gew.%
Li₂O 0 - 15 Gew%
Na₂O 5 - 40 Gew.%
K₂O 0-25 Gew.%
CaO 5 - 40 Gew.%
MgO 0 -15 Gew.%
SrO 0 - 15 Gew.%
BaO 0 - 15 Gew%
ZnO 0 - 40 Gew.%
Ag₂O 0 - 5 Gew.%
J 0 - 10 Gew.%
Fe₂O₃ 0 - 5 Gew.%.

9. Verwendung nach einem der vorhergehenden Ansprüche 1 bis 7, wobei die Glaszusammensetzung folgende Inhaltsstoffe aufweist
SiO₂ 0 - 10 Gew.%
P₂O₅ 25 - 75 Gew.%
SO₃ 0 - 30 Gew.%
B₂O₃ 0 - 40 Gew.%
Al₂O₃ 0 - 10 Gew.%
Li₂O 0 - 15 Gew%
Na₂O 0 - 40 Gew.%
K₂O 0 - 25 Gew.%
CaO 0 - 40 Gew.%
MgO 0-15 Gew.%
SrO 0 - 15 Gew.%
BaO 0 - 15 Gew%
ZnO 0 - 40 Gew.%
Ag₂O 0 - 5 Gew.%
J 0 - 10 Gew.%
Fe₂O₃ 0 - 5 Gew.%.

10. Verwendung nach Anspruch 9, wobei in der Glaszusammensetzung die Summe von CaO + MgO + SrO + BaO + ZnO von 0 bis 40 Gew.% beträgt.

11. Verwendung nach Anspruch 9 oder 10, wobei die Glaszusammensetzung alkalifrei ist.

12. Verwendung nach Anspruch 9 oder 10, wobei in der Glaszusammensetzung die Summe von Li₂O + Na₂O +K₂O von 5 bis 50 Gew.% beträgt.

13. Verwendung nach einem der vorhergehenden Ansprüche in Kosmetikprodukten.

14. Verwendung nach einem der vorhergehenden Ansprüche in deodorierenden Produkten.

15. Verwendung nach einem der vorhergehenden Ansprüche 1 bis 13 im Bereich der Mundhygiene, Zahnpflege, Mundpflege, Zahnfleischhygiene.

16. Verwendung nach einem der vorhergehenden Ansprüche 1 bis 13 in Kosmetikprodukten, die einer vorzeitigen Alterung der Haut vorbeugen sollen ("Anti-Aging" Cremes bzw. Antifaltencremes).

17. Verwendung nach einem der vorhergehenden Ansprüche 1 bis 12 zur Herstellung von Medizinprodukten, zum Erzielen eines antioxidativen Effekts.

18. Verwendung nach einem der vorhergehenden Ansprüche 1 bis 12 in Lebensmitteln.

19. Verwendung nach einem der vorhergehenden Ansprüche 1 bis 12 in Futtermitteln.

20. Verwendung nach einem der vorhergehenden Ansprüche 1 bis 12 in Farben und Lacken.

21. Verwendung nach einem der vorhergehenden Ansprüche 1 bis 11 in Putzen, Zementen und Beton.

22. Verwendung nach einem der vorhergehenden Ansprüche 1 bis 12 in Anti-Fouling Produkten.

23. Verwendung nach einem der vorhergehenden Ansprüche 1 bis 12 in Polymeren.

## Claims

1. Use of a glass composition for achieving an anti oxidative effect in the sense of a radical quencher or an anti oxidant.

2. Use according to claim 1, wherein the glass composition is oxidic.

3. Use according to one of the preceding claims, wherein the glass composition has high photo and temperature stability.

4. Use according to one of the preceding claims, wherein the glass composition comprises the following ingredients
SiO₂ 0 - 80 wt. %
P₂O₅ 0 - 80 wt. %
SO₃ 0 - 40 wt. %
B₂O₃ 0 - 80 wt. %
Al₂O₃ 0 - 20 wt. %
Li₂0 0 - 30 wt. %
Na₂0 0 - 40 wt. %
K₂O 0 - 30 wt. %
CaO 0 - 25 wt. %
MgO 0 -15 wt. %
SrO 0-15wt.%
BaO 0-15wt.%
ZnO 0 - 40 wt. %
Ag₂O 0 - 5 wt. %
F 0-10wt. %
J 0 - 10 wt. %
Fe₂O₃ 0 - 5 wt. %,
and optionally trace elements and/or common fining agents in common amounts, wherein the sum SiO₂ + P₂O₅ + SO₃ + B₂O₃ + Al₂O₃ exceeds 20 % by weight and amounts to up to 80 % by weight.

5. Use according to one of the preceding claims, wherein the glass composition is free of alkali oxides.

6. Use according to one of the preceding claims, wherein the sum CaO + MgO + SrO + BaO + ZnO in the glass composition is in a range from 5 to 50 % by weight.

7. Use according to one of claims 1 to 4 or 6, wherein the sum Li₂O + Na₂O +K₂O adds up to 5 to 60 % by weight, in the glass composition.

8. Use according to one of the preceding claims, wherein the glass composition comprises the following ingredients
P₂O₅ 1 - 15 wt. %
B₂O₃ 0-5wt. %
Al₂O₃ 0-10 wt. %
Li₂O 0-15wt. %
Na₂O 5-40 wt. %
K₂O 0 - 25 wt. %
CaO 5 - 40 wt. %
MgO 0 -15 wt. %
SrO 0 - 15 wt. %
BaO 0 - 15 wt. %
ZnO 0-40 wt. %
Ag₂O 0 - 5 wt. %
J 0-10wt.%
Fe₂O₃0-5wt %.

9. Use according to one of the preceding claims 1 to 7, wherein the glass composition comprises the following ingredients
SiO₂ 0-10wt.%
P₂O₅ 25 - 75 wt. %
SO₃ 0-30wt.%
B₂O₃ 0-40 wt. %
Al₂O₃ 0-10wt. %
Li₂O 0-15wt.%
Na₂O 0-40 wt. %
K₂O 0-25 wt. %
CaO 0-40 wt. %
MgO 0 - 15 wt.%
SrO 0 - 15 wt.%
BaO 0 - 15 wt. %
ZnO 0-40 wt. %
Ag₂O 0 - 5 wt. %
J 0-10wt.%%
Fe₂O₃ 0 - 5 wt. %%.

10. Use according to claim 9, wherein the sum CaO + MgO + SrO + BaO + ZnO adds up to 0 to 40 % by weight in the glass composition.

11. Use according to claim 9 or 10, wherein the glass composition is free of alkali.

12. Use according to claim 9 or 10, wherein the sum Li₂O + Na₂O +K₂O in the glass composition adds up to 5 to 50 % by weight.

13. Use according to one of the preceding claims in cosmetic products.

14. Use according to one of the preceding claims in deodorant products.

15. Use according to one of the preceding claims 1 to 13 in the fields of oral hygiene, dental care, oral care, gingival care.

16. Use according to one of the preceding claims 1 to 13 in cosmetic products for preventing skin aging (anti-aging creames and/or anti-wrinkle creams).

17. Use according to one of the preceding claims 1 to 12 for preparation of medical devices for achieving an anti oxidative effect.

18. Use according to one of the preceding claims 1 to 12 in foods.

19. Use according to one of the preceding claims 1 to 12 in animal feed.

20. Use according to one of the preceding claims 1 to 12 in paints and lacquers.

21. Use according to one of the preceding claims 1 to 11 in plasters, cements and concretes.

22. Use according to one of the preceding claims in anti fouling products.

23. Use according to one of the preceding claims 1 to 12 in polymers.

## Revendications

1. Utilisation d'une composition de verre pour obtenir un effet antioxydant, au sens d'un piégeur de radicaux (radical scavenger) ou d'un anti-oxydant.

2. Utilisation selon la revendication 1, dans laquelle la composition de verre est composé d'oxydes.

3. Utilisation selon l'une quelconque des revendications précédentes, dans laquelle la composition de verre présente une photorésistance élevée et une résistance thermique élevée.

4. Utilisation selon l'une quelconque des revendications précédentes, dans laquelle la composition de verre comporte les composants suivants
SiO₂ 0 - 80 % en poids
P₂O₅ 0 - 80 % en poids
SO₃ 0 - 40 % en poids
B₂O₃ 0 - 80 % en poids
Al₂O₃ 0 - 20 % en poids
Li₂O 0 - 30 % en poids
Na₂O 0 - 40 % en poids
K₂O 0 - 30 % en poids
CaO 0 - 25 % en poids
MgO 0-15% en poids
SrO 0-15% en poids
BaO 0-15% en poids
ZnO 0 - 40 % en poids
Ag₂O 0 - 5 % en poids
F 0 - 10 % en poids
J 0 - 10 % en poids
Fe₂O₃ 0 - 5 % en poids,
et, le cas échéant, peut contenir des oligo-éléments et/ou les quantités courantes de les agents d'affinage appropriés, la somme SiO₂ + P₂O₅ + SO₃ + B₂O₃ + Al₂O₃ étant supérieur à 20 % en poids jusqu'à 80 % en poids.

5. Utilisation selon l'une quelconque des revendications précédentes, dans laquelle la composition de verre ne contient pas les oxydes d'alcalis.

6. Utilisation selon l'une quelconque des revendications précédentes, dans laquelle la somme CaO + MgO + SrO + BaO + ZnO dans la composition de verre est de 5 à 50 % en poids.

7. Utilisation selon l'une quelconque des revendications 1 - 4 ou 6, dans laquelle la somme Li₂O + Na₂O +K₂O dans la composition de verre est de 5 à 60 % en poids.

8. Utilisation selon l'une quelconque des revendications précédentes, dans laquelle la composition de verre comporte les composants suivants
P₂O₅ 1 - 15 % en poids
B₂O₃ 0 - 5 % en poids
Al₂O₃ 0 - 10 % en poids
Li₂O 0 - 15 % en poids
Na₂O 5 - 40 % en poids
K₂O 0 - 25 % en poids
CaO 5 - 40 % en poids
MgO 0-15% en poids
SrO 0-15% en poids
BaO 0-15% en poids
ZnO 0 - 40 % en poids
Ag₂O 0 - 5 % en poids
J 0 - 10 % en poids
Fe₂O₃ 0 - 5 % en poids.

9. Utilisation selon l'une quelconque des revendications 1 - 7, dans laquelle la composition de verre comporte les composants suivants
SiO₂ 0 - 10 % en poids
P₂O₅ 25 - 75 % en poids
SO₃ 0 - 30 % en poids
B₂O₃ 0 - 40 % en poids
Al₂O₃ 0 - 10 % en poids
Li₂O 0 - 15 % en poids
Na₂O 0 - 40 % en poids
K₂O 0-25 % en poids
CaO 0-40% en poids
MgO 0-15% en poids
SrO 0-15% en poids
BaO 0-15% en poids
ZnO 0-40% en poids
Ag₂O 0-5% en poids
J 0-10% en poids
Fe₂O₃ 0 - 5 % en poids.

10. Utilisation selon de revendication 9, dans laquelle la somme CaO + MgO + SrO + BaO + ZnO dans la composition de verre est de 0 à 40 % en poids.

11. Utilisation selon de revendications 9 ou 10, dans laquelle la composition de verre ne contient pas d'alcali.

12. Utilisation selon de revendications 9 ou 10, dans laquelle la somme Li₂O + Na₂O +K₂O dans la composition de verre est de 5 à 50 % en poids.

13. Utilisation selon l'une quelconque des revendications précédentes dans les produits cosmétiques.

14. Utilisation selon l'une quelconque des revendications précédentes dans les produits déodorants.

15. Utilisation selon l'une quelconque des revendications 1-13 dans les domaines d'hygiène bucco-dentaire, l'hygiène dentaire, l'hygiène buccale, l'hygiène gingivale.

16. Utilisation selon l'une quelconque des revendications 1 - 13 dans les produits cosmétiques pour prévenir vieillissement cutané (crèmes anti-âge et/ou antirides).

17. Utilisation selon l'une quelconque des revendications 1-12 pour la fabrication de les produits médicaux, pour obtenir un effet antioxydant.

18. Utilisation selon l'une quelconque des revendications 1-12 dans les aliments.

19. Utilisation selon l'une quelconque des revendications 1-12 dans les aliments pour animaux.

20. Utilisation selon l'une quelconque des revendications 1 - 12 dans les couleurs et vernis.

21. Utilisation selon l'une quelconque des revendications 1 - 11 dans les crépis, céments et bétons.

22. Utilisation selon l'une quelconque des revendications 1 - 12, dans les produits antifouling.

23. Utilisation selon l'une quelconque des revendications 1 - 12 dans polymères.
